# EUROPEAN PATENT APPLICATION

(11) **EP 4 309 503 A1**
(43) Date of publication of application: **24.01.2024**
(21) Application number: 22186076.0
(22) Date of filing: 20.07.2022
(51) Int. Cl.: A21D 2/08, A23K 20/189, A23L 29/00, A61K 38/43, C12N 9/96

(54) **PROTEIN FORMULATION**

(71) Applicant: Kaesler Nutrition GmbH, 27472 Cuxhaven (DE)
(72) Inventor: DIETZ, Heiko, 27472 Cuxhaven (DE); GENTHE, Niclas, 27472 Cuxhaven (DE); SCHMIDT, Mario, 27472 Cuxhaven (DE); FISCHBECK, Kai Florian Sunim, 27472 Cuxhaven (DE)
(74) Representative: V.O.

(57) **Abstract**

The invention relates to a protein formulation comprising a protein, such as an enzyme, and a protective agent, said protective agent comprising (i) at least one amine and/or an ammonium group and (ii) at least one metal precipitating agent and/or a chelating molecule; the invention also relates to a method of making such protein formulation, and to the use of such protective agent for improving the stability of a protein formulation.

## Description

The invention relates to a protein formulation, a food product comprising a protein formulation and a method of preparing a protein formulation.

The use of proteins, in particular enzymes as active agents in the food industry is becoming increasingly popular, among others due to positive health effect on the gastro-intestinal tract of animals.

However, many proteins used for industrial processes suffer from insufficient stability against conditions typically employed in the manufacturing of protein formulations or food products comprising such protein formulations.

For example, many proteins are sensitive to heat and therefore at least partially degrade when heat is applied, for example during extrusion or steam pelletizing of the protein with other food ingredients. This is a disadvantage, since many types of food products, such as animal feed, are preferably formulated in the form of a pellet, for reasons of storage efficiency, stability and ease of handling.

This problem has been addressed by EP 2 497 372, wherein a granule is described comprising a core, an active agent such as an enzyme, and at least one protective coating. The coating aims to prevent migration of moisture into the core comprising the active agent. Typically, the coating comprises a moisture barrier coating that slows down the rate of moisture migration into the granule, and/or a moisture hydrating coating absorbing moisture, thereby impeding or retarding the extend or rate of transport of external moisture into the core.

However, such coated granules still suffer from substantial degradation after exposure to pelletizing at 90 °C and furthermore requires the additional step of applying a coating to a core comprising an enzyme.

The present inventors developed a protein formulation that overcomes one or more of the above-mentioned drawbacks. In particular, the present inventors developed a protein formulation that has similar or improved stability compared to prior art protein formulations, in particular similar or improved stability of the protein against heat and/or similar or improved storage stability. Said protein formulation comprises a protective agent, which can be mixed with a protein and optionally other components to obtain the protein formulation, without requiring a step of coating. This is advantageous, because the protein formulation can be prepared in a more time and cost efficient manner.

Accordingly, the invention relates to a protein formulation comprising a protein and a protective agent, said protective agent comprising (i) at least one amine and/or an ammonium group and (ii) at least one metal precipitating agent and/or a chelating molecule.

### Figures

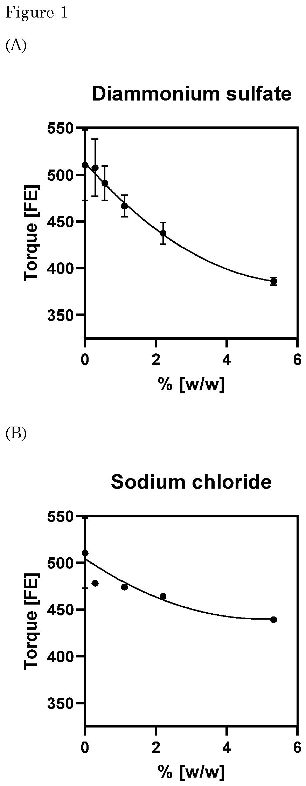

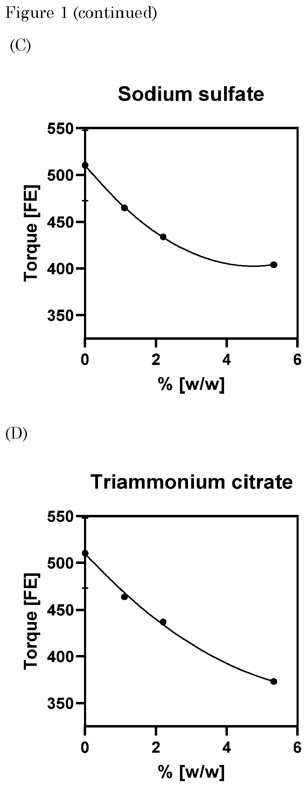

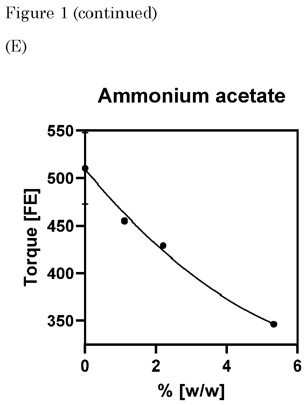

Figure 1: Power input required for mixing aqueous mixtures of wheat flour and increasing quantities of diammonium sulphate (A), sodium chloride (B), sodium sulphate (C), triammonium sulphate (D) and ammonium acetate (E). For each salt, the energy required for mixing was reduced at increasing quantities of salt.

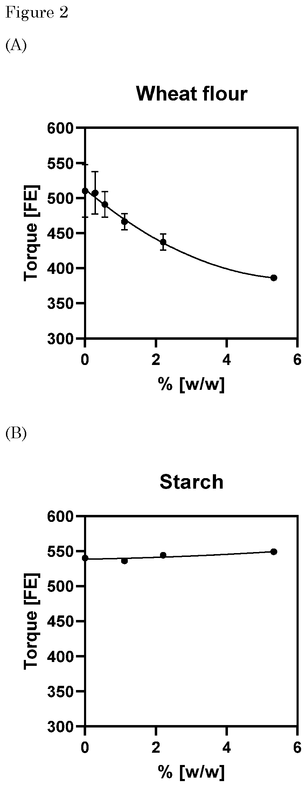

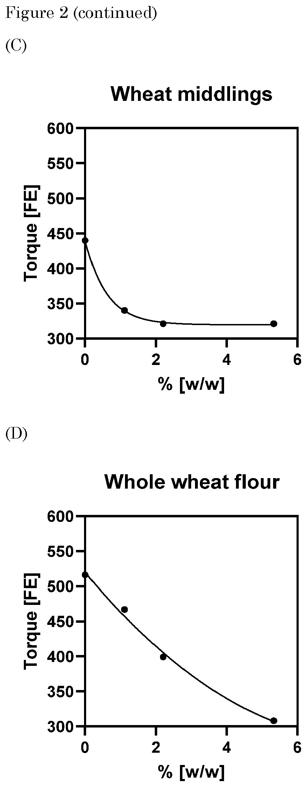

Figure 2: Power input required for mixing aqueous mixtures of wheat flour (A), starch (B), wheat midlings (C) and whole wheat flour (D) in presence of increasing quantities of diammonium sulphate. It is demonstrated that the energy required for mixing decreases in presence of gluten and an ammonium salt.

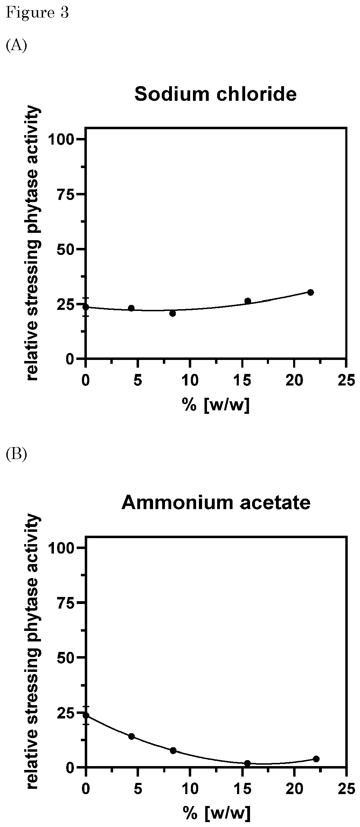

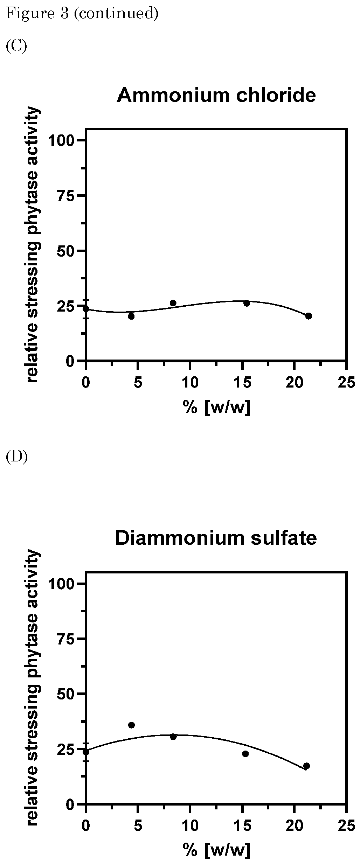

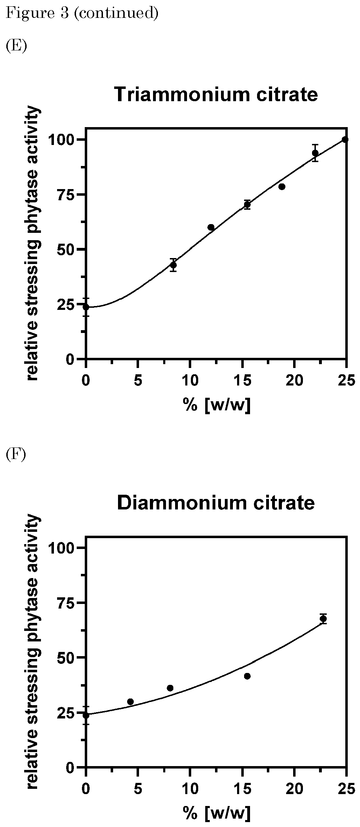

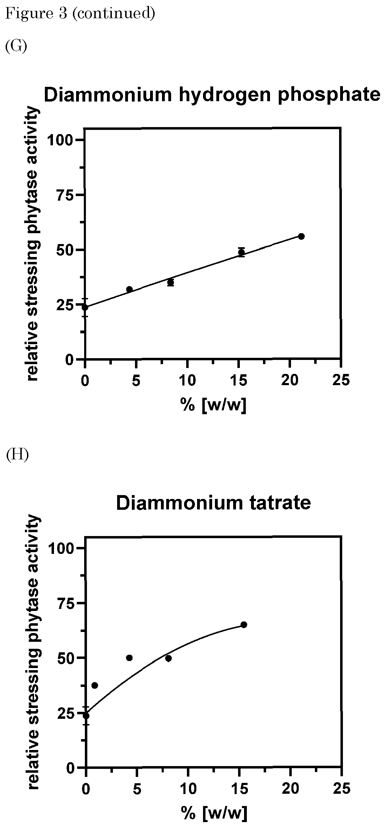

Figure 3: Relative enzyme activity after exposing phytase formulations to thermostability testing. It is demonstrated that the relative enzyme activity is not improved or even decreased in presence of sodium chloride (A), ammonium acetate (B), ammonium chloride (C) or ammonium sulphate (D), but is improved in presence of triammonium citrate (E), diammonium hydrogen citrate (F), diammonium hydrogen phosphate (G) and diammonium tartrate (H).

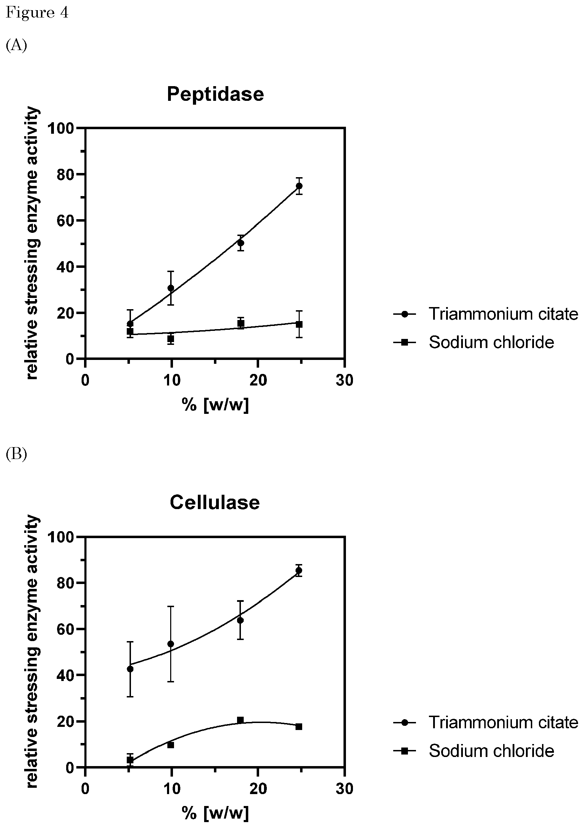

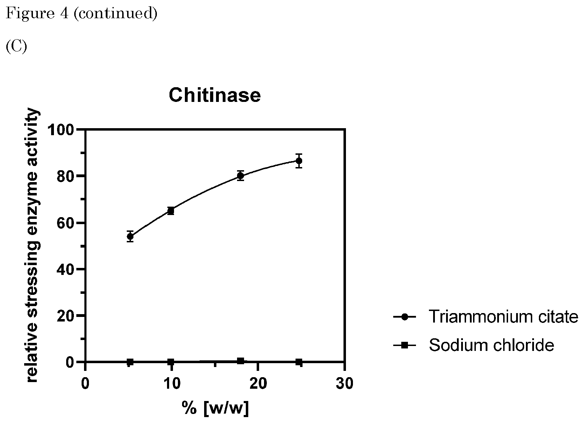

Figure 4: Relative enzyme activity after thermostability testing of a peptidase formulation (A), cellulase formulation (B) and chitinase formulation (C) in presence of sodium chloride or triammonium citrate. It is demonstrated that the enzyme activity after thermostability testing is improved in presence of triammonium citrate but not in presence of sodium chloride.

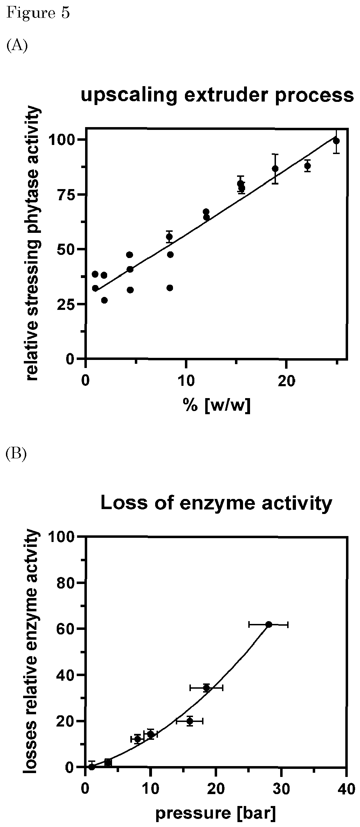

Figure 5: Relative phytase activity after exposing a phytase formulation at technical scale (A). Relative phytase activity loss when extrusion was carried out at increasing pressure (B). It is demonstrated that at higher pressure, more loss of phytase activity was observed.

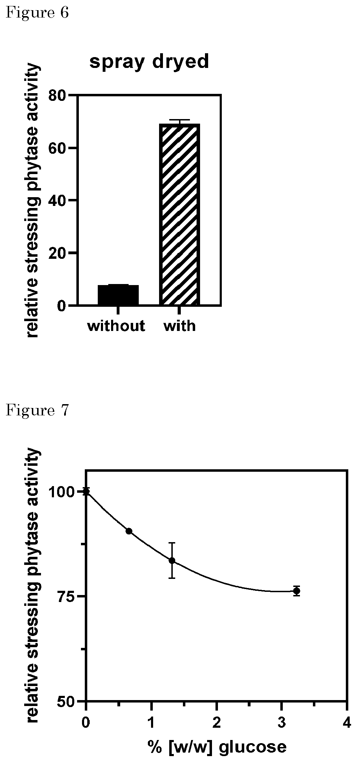

Figure 6: Relative phytase activity in a spray-dried phytase formulation both in presence (striped bar) and absence (black bar) of triammonium citrate. The results demonstrate that an enzyme formulation according to the invention can also be prepared by means of spray-drying.
Figure 7: Relative phytase activity in presence of increasing concentrations of glucose. It is demonstrated that the phytase activity after thermostability testing reduces at increasing concentrations of glucose.

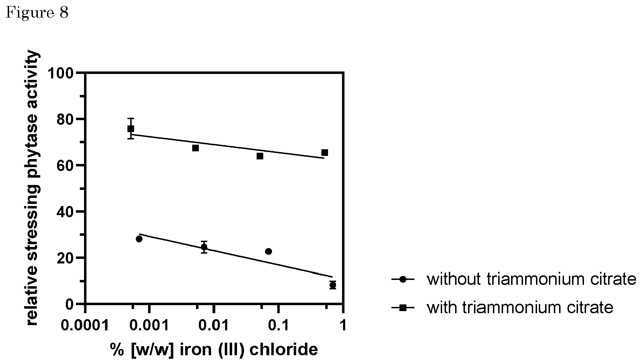

Figure 8: Relative phytase activity after thermostability testing of an enzyme formulation, both in presence of increasing concentration of iron(iii) chloride both in presence (squares) and absence (balls) of triammonium citrate. The results demonstrate that the phytase activity can be significantly improved in presence of triammonium citrate.

### Detailed description

The term 'or' as used herein is defined as 'and/or' unless specified otherwise.

The term 'a' or 'an' as used herein is defined as "at least one" unless specified otherwise.

When referring to a noun (*e.g.* a compound, an additive, *etc*.) in the singular, the plural is meant to be included.

The term "essential(ly) or "substantial(ly)" is generally used herein to indicate that it has the general character or function of that which is specified. When referring to a quantifiable feature, this term is generally used to indicate that it is more than 30%, in particular more than 50%, in particular more than 70%, more in particular at least 90%, more in particular at least 95%, even more in particular at least 98% of the maximum of that feature. The term 'essentially free' is generally used herein to indicate that a substance is not present (below the detection limit achievable with analytical technology as available on the effective filing date) or present in such a low amount that it does not significantly affect the property of the product that is essentially free of said substance.

In the context of this application, the term 'about' means generally a deviation of 15 % or less from the given value, in particular a deviation of 10% or less, more in particular a deviation of 5%, 4%, 3%, 2%, 1%, 0.5% or less.

A 'protein' as used herein refers to a chain of amino acids arranged in a specific order determined by the coding sequence in a polynucleotide encoding the polypeptide. Typically, the chain comprises at least 10 amino acids or more, preferably at least 15 amino acids or more, such as 20 amino acids or more. There is no maximum number of amino acids that may be present in a protein, although generally speaking, a protein has at most 40.000 amino acid residues, such as 35.000 amino acid residues. On average, a protein typically comprises about 100 and about 1500 amino acid residues.

An 'enzyme' as used herein refers to a protein that has an ability of catalysing a biochemical reaction under physiological conditions. Enzymes may be referred to herein by their corresponding Enzyme Commission (EC) numbers as determined by the nomenclature committee of the international union of biochemistry and molecular biology (NC-IUBMB) (https://iubmb.qmul.ac.uk/enzyme/index.html; accessed on 17 June 2022). Examples of enzymes include hydrolases, such as phosphatases and peptidases.

A 'reducing sugar' is generally known in the art to refer to a sugar molecule that, in open form, comprises an aldehyde group that has an activity of acting as a reducing agent. Typically, reducing sugars may be present both in closed form, wherein the reduced sugar forms a 5-membered or 6-membered ring having a hemiacetal or hemiketal functionality; and an open form, wherein the hemiacetal in the closed form is opened to form a linear structure comprising an aldehyde group at the terminal end. Examples of reducing sugars include glucose, fructose, lactose and maltose.

An 'amine' as used herein is generally understood in the art to refer to a functional group having the structural formula NR₁R₂R₃, wherein R₁, R₂ and R₃ may be the same or different and may be any group that renders a chemically stable molecule under physiological conditions. An example of an amine is NH₃, wherein each R₁, R₂ and R₃ represent a hydrogen group ('H').

An 'ammonium' as used herein is generally understood in the art to refer to a functional group having the structural formula N⁺R₁R₂R₃H, wherein R₁, R₂ and R₃ may be the same or different and may be any group that renders a chemically stable molecule under physiological conditions. An example of an ammonium is the NH₄⁺ ion, wherein each R₁, R₂, and R₃ represent a hydrogen group ('H').

A 'metal' as used herein generally means an element from group 1 to group 13 of the periodic table, except for hydrogen and boron, or an element selected from Tin (Sn), Lead (Pb), Bismuth (Bi) and Polonium (Po). Examples of metals include transition metals, alkali metals, alkaline earth metals, lanthanides and actinides.

With the term 'transition metal' as used herein is typically meant an element selected from group 3, period 4 and 5, group 4, periods 4 to 7, group 5 periods 4 to 7, group 6, periods 4 to 7, group 7, periods 4 to 7, group 8, periods 4 to 7, group 9, periods 4 to 6, group 10, periods 4 to 6, group 11, periods 4 to 6 and group 12, periods 4 to 7. Said transition metal may be in any oxidation state, but is preferably a divalent or trivalent metal ion.

A salt is defined as a compound that is formed by chemical combination of an acid and a base, or through neutralization. Salts may be formed when the ions are joined together by an ionic bond. A salt may dissociate into ions (other than H⁺ or OH⁻) when dissolved in a solvent such as water. An "inorganic salt" is generally understood in the art to refer to a salt that does not comprise a C-H bond in its scaffold. Examples of inorganic salts include sodium chloride, ammonium phosphate and the like. An "organic salt" is generally understood in the art to refer to a salt that is not inorganic, i.e. a salt that does comprise a C-H bond in its scaffold. Examples include salts of citrate or tartrate.

The term "nucleophile" or "nucleophilic group" is generally understood in the art to refer to a molecule or functional group that is capable of donating electrons by reacting with an electrophilic group. Typically, a nucleophile has a high electron density, e.g. due to the presence of a free electron pair (also referred to as lone electron pair) and/or an adjacent electron-donating group. Herein, an "electrophile" or "electrophilic group" is understood in the art to refer to a molecule or group that is capable of receiving electrons. Typically, an electrophile has a low electron density, e.g. due to the presence of an adjacent electron-withdrawing group. An "electron withdrawing" group is generally understood to refer to a group that is capable of reducing the electron density of an adjacent group, e.g. through polarization (induction) or stabilization by delocalization of electrons. An electron-donating group is generally understood in the art to refer to a group that is capable of increasing the electron density of an adjacent group, e.g. through polarization (induction) or delocalization of electrons.

In the context of the present application, the terms "chelating agent", "chelating molecule" or "chelator" are used interchangeably herein and generally refer to a molecule that has an ability of binding metal ions, preferably transition metal ions, thereby forming a complex. Typically a chelating molecule is a polydentate molecule, such as a bidentate molecule, comprising at least two functional groups that have an ability to coordinate a metal ion, preferably a transition metal ion, more preferably a divalent or trivalent transition metal ion. Examples of divalent transition metal ions (M²⁺) that may be coordinated by a chelating molecule include Cu²⁺, Co²⁺, Ni²⁺, Mn²⁺, Zn²⁺ and Fe²⁺. An example of a trivalent metal ion (M³⁺) is Fe³⁺. Examples of functional groups that have the ability to coordinate a transition metal ion include carboxylate groups and amines.

In the context of the present application, a "metal precipitating agent" generally means an agent that has an ability to form a complex or salt with a metal ion, preferably a transition metal ion, that is not soluble or has limited solubility in an aqueous medium at about 25 °C, atmospheric pressure and physiological pH. Solubility in an aqueous medium may be determined by the eye, e.g. by observing a precipitate, crystal, haze, turbidity or the like, or it may be detected using analytical means, such as using high-performance liquid chromatography.

As the skilled person will appreciate, in the context of the present application, said metal precipitating agent may form a salt or a complex with a metal ion, preferably a transition metal ion, with limited solubility in any suitable aqueous medium. Examples of suitable aqueous media include limited solubility in an aqueous solution (such as an aqueous enzyme solution), and limited solubility in an aqueous phase of an aqueous emulsion or aqueous suspension. An example of an aqueous suspension is a dough comprising protein, water and flour. Herein, limited solubility may refer to the limited solubility in an aqueous phase of the dough.

With the term 'dough' as used herein is typically meant a mixture comprising at least a flour, water, a protein and a protective agent, which has a viscoelastic consistency. A dough is typically kneadable using a suitable tool such as hands or a mechanical mixer. A dough is typically able to substantially hold its shape in absence of a tool holding it in place, e.g. a container.

In the context of the present application a "food product" refers to any product that is edible for animals, including humans, meaning that dietary intake is not advised against by common health authorities, for example the FDA and/or the EFSA.

### Protein formulation

It is believed that the instability of the protein, in particular instability of a protein against heat (typically a temperature of 40 °C or more) or over time (typically at least 24 hours) at ambient temperature, may be at least partly due to degradation caused by the presence of reducing sugars and/or metal ions, which are typically present in a protein formulation, e.g. as remnants from a protein production process.

Without wishing to be bound by any theory, it is envisaged that amines present in the amino acid residues of the protein may react with an aldehyde group of a reducing sugar. This is referred to in the art as a "Maillard reaction". (Kaufmann, 2018, PhD dissertation; 'Dynamik der Zuckertautomerie und ihr Einfluss auf die Kinetik der Maillard-reaktion").

Said Maillard reaction may be accelerated by metal ions, in particular transition metal ions (Kato et al., 1981. J. Agric. Food. Chem, 29, 540-543).

Accordingly, by introducing a protective agent in a protein formulation that at least partly removes reducing sugars and metal ions as active components from the protein formulation, the protein present in the formulation is prevented from participating into a Maillard reaction. This significantly improves the tolerance of a protein formulation to the presence of reducing sugars and metal ions, in particular transition metal ions, compared to other protein formulations known in the art. Thereby the stability of a protein over time and/or at elevated temperatures may be markedly improved.

Accordingly, the invention relates to a protein formulation comprising a protein and a protective agent, said protective agent comprising (i) at least one amine and/or an ammonium group and (ii) at least one metal precipitating agent and/or a chelating molecule.

### Protein

The protein formulation according to the invention may comprise any protein of interest, i.e. any protein that has a relevant commercial, nutritional, pharmaceutical or scientific use.

For example, the protein may be an antibody, a peptide, preferably a therapeutic peptide, such as an antimicrobial peptide or an enzyme.

Preferably, the protein is an enzyme. Preferably the protein is an enzyme that has value as a food ingredient in a food product, more preferably in animal feed, in the household industry, such as a detergent, in leather processing or in the fermentation of waste, such as textile waste, paper waste, waste water and the like.

Preferably the protein in a protein formulation according to the invention is an enzyme belonging to the class of oxidoreductases (EC1), transferases (EC2), hydrolases (EC3), lyases (EC4), isomerases (EC5), ligases (EC6) or translocases (EC7), most preferably, an enzyme in an enzyme formulation according to the invention belongs to the class of hydrolases (EC3).

The protein in a protein formulation according to the invention may be an enzyme from the class of oxidoreductases, such as an enzyme from subclass EC1.1, EC1.2, EC1.3, EC1.4, EC1.5, EC1.6, EC1.7, EC1.8, EC1.9, EC1.10, EC1.11, EC1.12, EC1.13, EC1.14, EC1.15, EC1.16, EC.1.17, EC1.18, EC1.19, EC1.20, EC1.21, EC1.22, EC1.23 or EC1.97.

Alternatively, the protein in a protein formulation according to the invention is an enzyme from the class of transferases, such as an enzyme from the subclass EC2.1, EC2.2, EC2.3, EC2.4, EC2.5, EC2.6, EC2.7, EC2.8, EC2.9 and EC2.10.

Preferably, the protein in a protein formulation according to the invention is an enzyme from the class of hydrolases, such as an enzyme from the subclass hydrolases acting on ester bonds (EC3.1), glycosylases (EC3.2), hydrolases acting on ether bonds (EC3.3), hydrolases acting on peptide bonds (EC3.4), hydrolases acting on carbon-nitrogen bonds, other than peptide bonds (EC3.5), hydrolases acting on acid anhydrides (EC3.6), hydrolases acting on carbon-carbon bonds (EC3.7), hydrolases acting on halide bonds (EC3.8), hydrolases acting on phosphorus-nitrogen bonds (EC3.9), hydrolases acting on sulfur-nitrogen bonds (EC3.10), hydrolases acting on carbon-phosphorus bonds (EC3.11), hydrolases acting on sulfur-sulfur bonds (EC3.12) and hydrolases acting on carbon-sulfur bonds (EC3.13).

Most preferably, the protein is an enzyme from the subclass EC3.1, EC 3.2 or EC 3.4.

Examples of enzymes from subclass EC 3.1 include carboxylic acid hydrolases (EC 3.1.1), thioester hydrolases (EC 3.1.2), phosphoric monoester hydrolases (EC 3.1.3), phosphoric diester hydrolases (EC 3.1.4), triphosphoric monoester hydrolases (EC 3.1.5), sulfuric ester hydrolases (EC 3.1.6), diphosphoric monoester hydrolases (EC 3.1.7), phosphoric trimester hydrolases (EC 3.1.8), Exodeoxyribonucleases Producing 5'-Phosphomonoesters (EC 3.1.11), Exodeoxyribonucleases Producing 5'-Phosphomonoesters (EC 3.1.12), Exoribonucleases Producing 5'-Phosphomonoesters (EC 3.1.13), Exoribonucleases Producing 3'-Phosphomonoesters (EC 3.1.14), Exonucleases Active with either Ribo- or Deoxyribonucleic Acids and Producing 5'-Phosphomonoesters (EC 3.1.15), Exonucleases Active with either Ribo- or Deoxyribonucleic Acids and Producing 3'-Phosphomonoesters (EC 3.1.16), Endodeoxyribonucleases Producing 5'-Phosphomonoesters (EC 3.1.21), Endodeoxyribonucleases Producing 3'-Phosphomonoesters (EC 3.1.22), Site-Specific Endodeoxyribonucleases Specific for Altered Bases (EC 3.1.25), Endoribonucleases Producing 5'-Phosphomonoesters (EC 3.1.26), Endoribonucleases Producing 3'-Phosphomonoesters (EC 3.1.27), Endoribonucleases Active with either Ribo- or Deoxyribonucleic Acids and Producing 5'-Phosphomonoesters (EC 3.1.30) and Endoribonucleases Active with either Ribo- or Deoxyribonucleic Acids and Producing 3'-Phosphomonoesters (EC 3.1.31).

Examples of enzymes from subclass EC 3.2 include glycosidases (EC 3.2.1), or hydrolyzing *N*-Glycosyl Compounds (EC 3.2.2).

Examples of enzymes from subclass EC 3.4 include Aminopeptidases (EC 3.4.11), Dipeptidases (EC 3.4.13), Dipeptidyl-peptidases and tripeptidyl-peptidases (EC 3.4.14), Peptidyl-dipeptidases (EC 3.4.15), Serine-type carboxypeptidases (EC 3.1.16), Metallocarboxypeptidases (3.4.17), Cysteine-type carboxypeptidases (3.4.18), Omega peptidases (3.4.19), Serine endopeptidases (3.4.21), cysteine endopeptidases (EC 3.4.22), aspartic endopeptidases (EC 3.4.23), metalloendopeptidases (EC 3.4.24), threonine endopeptidases (EC 3.4.25), endopeptidases of unknown catalytic mechanism (EC 3.4.99).

Most preferably, the protein in a protein formulation according to the invention is a 3-phytase (EC 3.1.3.8), a 4-phytase, also referred to in the art as 6-phytase (EC 3.1.3.26), a 5-phytase (EC 3.1.3.72), a chitinase (EC 3.2.1.14), a cellulase (EC 3.2.1.4), a peptidases (EC 3.4), a xylanase (EXC 3.2.1), an amylase (EC 3.2.1), a lipase (EC 3.1.1), a mannanase (EC 3.2.1), a pectinase (EC 3.2.1.15, a β-glucanase (EC 3.2.1.6), an α-galactosidase (EC 3.1.2.22).

In an aspect, the protein in a protein formulation according to the invention is an enzyme from the class of lyases, such as an enzyme from the subclass EC4.1, EC4.2, EC4.3, EC4.4, EC4.5, EC4.6, EC4.7 and EC4.99.

Alternatively, the protein in a protein formulation according to the invention is an enzyme from the class of isomerases, such as an enzyme from the subclass EC5.1, EC5.2, EC5.3, EC5.4, EC5.5 and EC5.99.

Alternatively, the protein in a protein formulation according to the invention is an enzyme from the class of ligases, such as an enzyme from the subclass EC6.1, EC6.2, EC6.3, EC6.4, EC6.5 and EC6.6.

The protein in a protein formulation according to the invention may be an enzyme from the class of translocases, such as an enzyme from the subclass EC7.1, EC7.2, EC7.3, EC7.4, EC7.5 and EC7.6.

The protein in a protein formulation according to the invention may be produced in any manner known in the art. Preferably, the protein, preferably the enzyme, is expressed by a suitable micro-organism in a medium or cell and subsequently isolated from said medium or cell. Micro-organisms that are suitable for expression of the protein, preferably enzyme, include filamentous fungi, yeasts, bacteria and algae. Said micro-organism may be a wildtype micro-organism or may be genetically modified, for example to improve the yield of the protein present in a protein formulation according to the invention.

During the process of expression of a protein, the protein, preferably the enzyme is usually expressed in a medium or in a cell which may be processed afterwards to obtain the protein from said medium or cell. The obtained protein, preferably enzyme, may therefore be obtained as a mixture comprising one or more further components originating from the medium or cell of the micro-organism, such as other proteins expressed by said micro-organism, peptides, amino acids, nucleic acids such as DNA or RNA, remnants of a cell of the protein-producing micro-organism, carbohydrates and lipids.

The obtained protein, preferably enzyme, may be at least partly purified to remove one or more of these components or a crude protein mixture may be used directly without substantial purification in a protein formulation according to the invention. Examples of purification include filtration, centrifugation and column chromatography, including combinations thereof.

An example of a commercially available 6-phytase suitable for use in the present invention includes Enzy Phostar, Kaesler Nutrition GmbH.

Preferably, a protein formulation according to the invention comprises remnants from a protein-producing micro-organism, more preferably said remnants include cellular matter, such as carbohydrates, including reducing sugars, lipids, including phospholipids and glycolipids or proteins.

Preferably a protein formulation according to the invention comprises one or more reducing sugars, such as galactose, glucose, mannose, fructose, rhamnose, galactosamine, xylose or sialic acid. Preferably said one or more reducing sugars comprises one or more of glucose, galactose, maltose, rhamnose and fructose.

In a preferred embodiment, a protein formulation according to the invention comprises one or more reducing sugars that is/are produced by a micro-organism, in particular a micro-organism that has expressed the protein present in said protein formulation or wherein the one or more reducing sugars is/are present as remnant(s) from a culture medium.

Said protein formulation preferably comprises cellular matter of said protein-producing micro-organism, such as remnants of said micro-organism and/or compounds excreted by said micro-organism. With the term "remnant" as used herein, is meant a compound which is generated or present during the protein-production process, including derivatives of such compounds. Included in the term "protein-production process" are any steps relating to at least partly isolating said protein from a cell or medium, such as extraction of a protein from a culture medium or cell.

Preferably, a protein formulation according to the invention comprises compounds present in the cell wall of a micro-organism, such as the cell-wall of a bacterium, yeast, fungus or algae, such as a glycoprotein, chitin or a glucan, or derivatives of such compounds, including breakdown products thereof.

In another preferred aspect, a protein formulation according to the invention comprises compounds present in the cell membrane of a micro-organism, such as the cell membrane of a bacterium, fungus, yeast or algae, including phospholipids and proteins, including fragments thereof.

Further preferred in a protein formulation according to the invention are compounds present in the cytoplasm of a micro-organism, including nucleic acid molecules such as plasmids, DNA and RNA ribosomes, intracellular membranes, proteins, including enzymes, lipids and carbohydrates.

In another preferred aspect, a protein formulation according to the invention comprises compounds excreted by a micro-organism, such as proteins, e.g. host-cell proteins and derivatives thereof, such as peptides and amino acids.

A protein formulation according to the invention, further preferably comprises remnants of a culture medium.

Examples of such compounds include a nitrogen source, e.g. peptone, glutamine or nitrates, a carbon source, such as a fermentable carbon source, e.g. glucose, lactose, molasse, corn steep liquor, growth factors, such as vitamins, minerals, such as calcium, potassium, magnesium, metal ions such as Zn²⁺, Cu²⁺, Fe²⁺ or Fe³⁺ and other breakdown products of these compounds.

Removing remnants from a fermentation or a culture medium from a protein requires one or more purification steps and may be challenging, especially to remove trace amounts of such remnants. In addition, purification steps typically result in a lower yield of a protein, as typically during purification at least a part of said protein is lost. It is thus an advantage of a protein formulation according to the invention, that certain amounts of fermentation remnants, in particular metal ions, such as transition metal ions and reducing sugar, is tolerated. This allows the use of a crude protein or a partially purified protein in a protein formulation according to the invention. Thus, it is not required to substantially purify the protein prior to its addition into a protein formulation according to the invention, thereby saving time, costs and avoiding undesired losses.

As the skilled person will appreciate said protein formulation meets the legal requirements set by relevant authorities, such as the Food and Drug Administration (FDA) or the European Food Safety Authority (EFSA) in case of a food product. Hence, if cellular matter, fermentation remnants or other compounds are present, they are present in a relatively low amount, such that the protein formulation meets the legal requirements defined by the relevant authorities.

Alternatively or additionally, said reducing sugars and/or metal ions may be added to a protein in a protein formulation, e.g. in the form of a carrier or an excipient. Examples of a carrier include flour, in particular wheat flour and lactose.

The protein may be added in any form to a protein formulation according to the invention, for example in dry form, e.g. a powder or a granulate, or in a solution.

Preferably, said protein is added in a solution, in particular in an aqueous solution. Said aqueous solution may comprise one or more further components for example to stabilize the protein in solution. Preferably, said aqueous solution comprises a stabilizer, a solubilizer, a cofactor or a co-solvent. Preferably, said aqueous solution further comprises glycerol. Glycerol may advantageously stabilize a protein by preventing aggregation during refolding of proteins.

Preferably, said protein is present in a protein formulation according to the invention in an amount of about 0,001 wt.% to about 50 wt.%, preferably between about 0,01 wt.% to about 40 wt.%, between about 0,1 wt.% and about 25 wt.%, most preferably between about 0,2 wt.% and about 10 wt.%, based on the total dry weight of the protein formulation.

### Protective agent

A protein formulation according to the invention further comprises a protective agent comprising (i) at least one amine and/or an ammonium group and (ii) at least one metal precipitating agent and/or a chelating molecule.

Without wishing to be bound by any theory, it is believed that said amine or ammonium group of the protective agent may react with an reducing sugar in a Maillard or Maillard-type reaction (Sengar and Sharma, J Food Sci Technol. 2014. 51(9):1686-1696). Thereby, any reducing sugar that may be present in a protein formulation is deactivated and the protein can therefore no longer, or at least to a lower extend, engage into a Maillard reaction, ultimately leading to degradation of said protein.

As the skilled person will appreciate, said protective agent may be present in a protein formulation according to the invention in the form of a molecule comprising an amine group. In this form, said protective agent may comprise a molecule represented with the chemical formula NR₁R₂R₃. In this form, said amine group comprises a lone electron pair capable of reacting as a nucleophile with the aldehyde of a reducing sugar.

Alternatively or additionally, said protective agent may be present in a protein formulation according to the invention in the form of an ammonium group. In this form, said protective agent may comprise a molecule represented with the chemical formula NR₁R₂R₃H⁺X⁻. In this form, said ammonium group may be engaged into a reversible reaction with the amine group. Generally, such a reversible reaction may be represented by the formula: HA + NR₁R₂R₃ 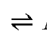 A⁻ + NR₁R₂R₃H⁺, wherein HA represents an acidic group capable of donating a H+ ion, and A- represents a basic group capable of receiving a H+ ion. As the skilled person will appreciate, the species A- does not necessarily have to carry a net negative charge, but may be represented by any species capable of receiving a H+ ion (e.g. a carbonyl or alcohol group present in a reducing sugar). Similarly, the species HA does not necessarily have to be neutral, but may be represented by any species capable of donating a H⁺ ion, such as an ammonium ion or ammonium salt.

Accordingly, without wishing to be bound by any theory, it is believed that when the protective agent comprises an ammonium group, it may generate the corresponding amine *in situ*, which may then be involved in the Maillard-type reaction as explained herein above. However, as the skilled person would understand, the electrons present in the N-H bond may also directly react with a carbonyl group, without generating an amine as intermediate species.

In the general formula's NR₁R₂R₃ and NR₁R₂R₃H⁺ X⁻, said substituents R₁, R₂ and R₃ may in principle be any substituent that renders a chemically stable molecule.

For example, substituents R₁, R₂ and R₃ may be each independently selected from the group consisting of a hydrogen (H), an alkyl group, an alkenyl group, an alkynyl group or an aryl group.

Said alkyl group may be a linear alkyl group, a branched alkyl group and is typically represented with the general formula CₙH₂ₙ₊₁, wherein n may be any integer, preferably an integer selected from 1, 2, 3, 4, 5, 6, 7, 8, 9, 10, 11 or 12. Preferably, said alkyl group is a methyl, an ethyl, a propyl, an isopropyl or a butyl group.

Said alkyl group may also be a cyclic group, typically represented with the general formula CₙH₂ₙ, wherein n may be any integer, preferably an integer selected from 3, 4, 5, 6, 7, 8, 9, 10, 11 or 12. Examples of cyclic alkyl groups include pentyl, hexyl and heptyl groups.

Said alkenyl group refers to any unsaturated aliphatic group comprising at least one double bound in its scaffold. Said alkenyl group may thus comprise one double bound, or multiple double bonds such as 2, 3, 4, 5, 6, 7, 8, 9, 10, 11 or 12 double bonds. Said alkenyl may be linear or branched. Examples of alkenyl groups include ethenyl, propenyl, butenyl and the like.

Said alkynyl group refers to any unsaturated aliphatic hydrocarbon comprising at least one triple bond in its scaffold. Said alkynyl group may thus comprise one triple bond, or multiple triple bonds such as 2, 3, 4, 5, 6, 7, 8, 9, 10, 11 or 12 triple bonds. Examples of alkenyl groups include ethynyl, propynyl, butynyl and the like.

Said aryl group refers to any group comprising an aromatic functionality in its scaffold. An aromatic group, as is known in the art, refers to a planar, cyclic group comprising unsaturated bonds, wherein the unsaturated bonds are fully conjugated, meaning that electrons can move freely through the p-orbitals present in the aromatic ring. A group or molecule is typically aromatic if it has 4n+2 π-electrons also referred to in the art as 'Hückels rule'. Said aryl group may comprise one aromatic group or multiple, such as two, three, four or five aromatic groups. Examples of aromatic groups include benzyl, phenyl, naphthyl or imidazole.

Herein, said X⁻ may be represented by any suitable negatively charged ion or molecule that is capable of forming an ionic bond with said positively charged NR₁R₂R₃H⁺ species. Preferably herein, X⁻ represents an organic acid, more preferably an organic acid selected from the group consisting of citrate, malate, malonate, oxalate, tartrate, lignosulfonate, humate, fulvate, pectin, amidated pectin, lactate, and urate.

Alternatively, X⁻ may represent an inorganic molecule, preferably a metal ion precipitating group, most preferably a phosphate group.

Preferably said substituents R₁, R₂ and R₃ are independently selected from hydrogen or a hydrocarbon. Alternatively, said substituent may comprise one or more functional groups, such as a carbonyl, an ester, a carboxylic acid, a halogen, an amine, an amide, a nitrile, a nitro group or an ether group.

As the skilled person will understand the substituents R₁, R₂ and R₃ at least partly determines the nucleophilicity and basicity of said amine group.

For example, when R₁, R₂ or R₃ represents an electron-donating group, the nucleophilic character of the amine group is enhanced, due to an increase of electron density of the lone electron pair present on the amine group. Examples of electron-donating groups include alkyl-groups, such as a linear alkyl groups including methyl, ethyl, propyl or isobutyl groups and cyclic alkyl groups such as a pentyl or hexyl group and aromatic groups such as a benzyl or a phenyl group.

Further, when R₁, R₂ or R₃ represents an electron-withdrawing group, the nucleophilic character of the amine group is decreased, due to a decrease of electron density of the lone pair present on the amine group. An example of an electron-withdrawing group is a carbonyl group.

Further, as the skilled person is aware, the presence of one or more substituents R₁, R₂ and R₃ increases the basicity of the amine compared to an amine lacking said substituents and decreases the nucleophilicity of the amine group compared to an amine lacking said substituents, due to steric hindrance caused by the presence one or more substituent R₁, R₂ and R₃.

Accordingly, preferably at least one of R₁, R₂ and R₃ in the general formula NR₁R₂R₃ is a hydrogen atom, more preferably at least two of R₁, R₂ and R₃ is a hydrogen atom, most preferably all of R₁, R₂ and R₃ are hydrogen atoms.

When one of R₁, R₂ and R₃ in the general formula NR₁R₂R₃ is a hydrogen atom, the general formula is NR₁R₂H, wherein R₁ and R₂ are as defined herein above, with the proviso that they are not hydrogen. This is referred to in the art as a secondary amine.

When two of R₁, R₂ and R₃ in the general formula NR₁R₂R₃ are hydrogen atoms, the general formula is NR₁H₂, wherein R₁ is as defined herein above, with the proviso that it is not a hydrogen atom. This is referred to in the art as a primary amine.

When all three of R₁, R₂ and R₃ in the general formula NR₁R₂R₃ are hydrogen atoms, the general formula is NH₃, also referred to in the art as ammonia. Preferably herein, the protective agent comprises ammonia.

In an aspect, the protective agent comprises an amino acid, in particular an amino acid selected from the group consisting of methionine, phenylalanine, tryptophan, asparagine, glutamine, serine, threonine, cysteine, lysine, arginine and histidine or a conjugate acid thereof.

Preferably, the protective agent in a protein formulation according to the invention comprises ammonia (NH₃), an ammonium ion (NH₄⁺) or both. If the protective agent comprises an ammonium ion, said ammonium ion is generally present in the form of a salt, having a negatively charged counterion or negatively charged molecule (X⁻).

Preferably, a protective agent in a protein formulation according to the invention comprises an ammonium salt of an organic acids, preferably an ammonium salt of citrate, malate, malonate, oxalate, tartrate, lactate, urate, lignosulfonate, humate, fulvate, pectin, and amidated pectin, or an ammonium salt of an inorganic salt, preferably phosphate, carbonate and sulfide.

Said protective agent further comprises at least one metal precipitating agent and/or a chelating molecule.

Preferably, said metal precipitating agent and/or a chelating molecule is capable of binding a transition metal ion, more preferably a transition metal of period 4 or period 5 of the periodic table, even more preferably a transition metal ion of period 4 of the periodic table.

Said transition metal is preferably a divalent or trivalent transition metal ion.

Said transition metal is preferably selected from scandium, titanium, vanadium, chromium, manganese, iron, cobalt, nickel, copper, zinc. Preferably, said transition metal ion is selected from Cu²⁺, Fe²⁺, Fe³⁺, Zn²⁺, Mn²⁺ or Mb²⁺ species. These are transition metal ions that are frequently present in protein formulations, in particular as remnants from a culture medium used to grow a micro-organism for producing a protein.

Preferably, said protective agent comprises a chelating molecule, more preferably a metal ion chelating molecule, in particular a transition metal ion chelating molecule, specifically a Fe³⁺, Fe²⁺, Cu²⁺, Mn²⁺, Mb²⁺ and/or Co²⁺ chelating molecule.

Without wishing to be bound by any theory, it is believed that chelating molecules advantageously form a stable, soluble, complex with a metal ion, preferably a transition metal ion. Thereby, said metal ion is prevented from accelerating a Maillard reaction, which leads to degradation of a protein in a protein formulation.

Preferably, the protective agent comprises citrate or tartrate, more preferably an ammonium salt of citrate, including diammonium hydrogen citrate, diammonium tartrate, ammonium hydrogen tartrate, ammonium dihydrogen citrate and triammonium citrate. Most preferably the protective agent comprises a triammonium citrate or diammonium hydrogen citrate.

Alternatively or additionally, said protective agent comprises a metal precipitating agent, preferably a metal ion precipitating agent, most preferably a transition metal ion precipitating agent, in particular a Fe³⁺, Fe²⁺, Cu²⁺, Mn²⁺, Mb²⁺, Co²⁺ precipitating agent.

Said metal precipitating agent preferably has an ability to form a complex or salt with a transition metal ion, which has limited solubility in an aqueous medium, such as a dough. Without wishing to be bound by any theory, it is believed that a metal precipitating agent has the ability to form a stable complex or salt with a metal ion with limited solubility in an aqueous medium, by preventing said metal ion from diffusing through the aqueous medium, such as the dough.

Consequently, the undesired effect of transition metal ions on enhancing the Maillard reaction is prevented, because said transition metal ions are removed from the reaction.

Preferably, said metal precipitating agent-metal ion complex or salt has a solubility in water of at most 10.000 µg/l, preferably 5000 µg/l, more preferably at most 1000 µg/l, more preferably 500 µg/l, more preferably at most 100 µg/l, at most 80 µg/l, at most 60 µg/l, at most 40 µg/l, most preferably at most 25 µg/l at 25 °C, atmospheric pressure and physiological pH.

Preferably, said salt or complex of a metal precipitating agent and transition metal ion has a solubility of between about 5 and about 10000 µg/l, such as between about 10 and about 1000 µg/l, preferably between about 15 and about 100 µg/l, between about 20 and about 50 µg/l, most preferably between about 21 and about 40 µg/l at 25 °C, atmospheric pressure and physiological pH.

Typically, with physiological pH is meant herein a pH around 7.4, preferably between about 4 and about 10, preferably between about 5 and about 9, between about 6 and about 8, most preferably around 7, in particular between 7 and 8.

As the skilled person will appreciate, solubility in water is a property of the metal precipitating agent-metal ion complex, which can be typically found in a suitable substance database, such as the "Gestis-Stoffendatenbank", https://gestis.dguv.de/list (accessed on 20 July 2022).

Experimentally, solubility in water can be determined using routine methods known in the art. For example, solubility can be determined by mixing an increasing amount of a component of which solubility is to be determined in water at 25 C, atmospheric pressure and physiological pH, until a precipitate, haze, turbidity or the like is observed, which indicates the maximum solubility in water has been found.

Solubility may also be determined using analytical means, such as using high-performance liquid chromatography.

Preferably, said metal precipitating agent is diammonium hydrogen phosphate, ammonium phosphate, ammonium dihydrogen phosphate, ammonium carbonate, ammonium hydrogen carbonate, ammonium hydrogen sulfide or ammonium sulfide.

Preferably, said metal precipitating agent is not a salt of sulfate or chloride.

As the skilled person will appreciate, said at least one amine and/or ammonium group may be comprised within the same molecule as said at least one metal precipitating agent and/or a chelating molecule.

Accordingly, the invention preferably relates to a protein formulation comprising a protein, preferably an enzyme, and a protective agent, wherein said protective agent is a molecule, preferably a salt, comprising (i) at least one amine and/or an ammonium group and (ii) at least one metal precipitating agent and/or a chelating group.

Preferably said molecule is a salt, comprising a cation and an anion, wherein said cation comprises ammonium and wherein said anion is a chelator or a metal precipitating agent.

Preferred examples of such a molecule include triammonium citrate, diammonium hydrogen citrate, ammonium dihydrogen citrate, diammonium tartrate, ammonium hydrogen tartrate, ammonium phosphate, ammonium hydrogen phosphate and diammonium hydrogen phosphate.

Alternatively, said protective agent may comprise at least two molecules, wherein a first molecule comprises at least one amine and/or ammonium group and a second molecule comprises at least one metal precipitating agent and/or a chelating molecule. For example, said protective agent may comprise a first molecule comprising an amine or ammonium group, and a second chelating molecule, such as citric acid or a salt thereof. Herein, said salt of citric acid may be an ammonium salt, or a different salt such as a sodium salt.

As the skilled person will appreciate, a protective agent that is formed *in situ* are also encompassed in the present invention.

In a protein formulation according to the invention, the w/w ratio between protein, preferably enzyme, and protective agent is preferably between about 1:5, preferably between about 1:10, between about 1:20, preferably between about 1:30, preferably between about 1:50, more preferably between 1:100.

The invention further preferably relates to a protein formulation that is at least partly tolerant against reducing sugars and/or metal ions, heat stable and/or storage stable. Preferably, at least 20% of the protein present in a protein formulation according to the invention is still active after said protein formulation has been subjected to a temperature of at least 50 °C. Most preferably at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, most preferably 100% of the protein is still active after a protein formulation according to the invention has been subjected to a temperature of at least 50 °C.

Activity of a protein can be determined using an appropriate activity assay, for example as demonstrated in Examples 3 and 4. For testing the activity of 6-phytase, the protocol according to ISO method 30024:2009 may for example be employed.

Preferably, a protein formulation according to the invention is stable at a temperature of at least 55 °C, at least 60 °C, at least 65 °C, at least 70 °C, at least 75 °C, at least 80 °C, at least 85 °C, at least 90 °C, at least 95 °C, at least 100 °C, at least 105 °C, at least 110 °C, at least 110 °C, most preferably at least 120 °C. These are conditions typically employed during extrusion or steam pelleting processes.

Preferably, a protein formulation according to the invention is stable at a temperature of between 55 °C and 150 °C, more preferably between 60 °C and 140 °C, between 65 °C and 130 °C, between 70 °C and 125 °C, between 75 °C and 120 °C, between 80 °C and 115 °C, between 85 °C and 110 °C, between 90 °C and 105 °C, between 95 °C and 100 °C.

Preferably at such temperatures, at least at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, most preferably 100% of the enzyme is still active. In a specific embodiment, at least 50%, preferably at least 60% of the enzyme is still active after a protein formulation according to the invention has been subjected to a temperature of at least 90 °C for at least 2 minutes, preferably at least 15 minutes.

Typically, a protein formulation according to the invention is subjected to heat, e.g. at least 50 °C, preferably at least 90 °C for a duration of about 10 seconds to about 5 hours, preferably between about 20 seconds to about 3 hours, between about 30 seconds to about 2 hours, between about 45 seconds to about 1 hours, between about 1 minute to about 45 minutes, between about 1.5 minute and about 30 minutes, most preferably between about 2 minutes and about 15 minutes.

The protective agent may be present in a protein formulation according to the invention in any amount that is sufficient to enhance the heat stability of the protein.

Preferably, the protective agent is present in an amount of between about 5 wt.% and about 50 wt.%, preferably between about 10 wt.% and about 40 wt.%, most preferably between about 15 wt.% and about 30 wt.%, based on dry weight of the protein formulation.

Preferably, the protective agent is present in an amount of at least 5 wt.% based on the dry weight of the protein formulation, more preferably at least 10 wt.%, at least 15 wt.%, at least 20 wt.%, at least 25 wt.% at least 30 wt.%, at least 35 wt.%, at least 40 wt.%, at least 45 wt.%, most preferably at least 50 wt.%, based on dry weight of the protein formulation.

Preferably, the protective agent is present in an amount of at most 50 wt.% based on the dry weight of the protein formulation, more preferably at most 45 wt.%, at most 40 wt.%, at most 35 wt.%, at most 30 wt.% at most 25 wt.% based on dry weight of the protein formulation.

A protein formulation according to the invention may further comprise one or more carriers. In principle, any carrier is suitable that is compatible with relevant regulations for protein formulations are suitable for use in a protein formulation according to the invention. Preferably, a protein formulation according to the invention comprises a flour, most preferably flour containing gluten, such as wheat flour, barley flour, rye flour, spelt flour, triticale flour and combinations thereof. Most preferably, a protein formulation according to the invention comprises wheat flour.

Such a protein formulation is particularly suitable for extrusion, because the protein formulation forms a dough, at least partly due to the presence of gluten in the flour. Such a dough may be advantageously subjected to extrusion, (steam) pelletizing or tableting processes, as described herein below.

Preferably, a protein formulation comprising flour, comprises at least 10 wt.% of flour, more preferably at least 20 wt.%, at least 30 wt.% at least 40 wt.%, at least 50 wt.% of flour, based on the total dry weight of the protein formulation.

Preferably, a protein formulation comprising flour comprises between about 10 and about 90 wt.% of flour, more preferably between about 20 wt.% and about 80 wt.% of flour, between about 30 wt.% and about 60 wt.% of flour, between about 40 wt.% and about 50 wt.% of flour, based on the total dry weight of the protein formulation.

Preferably, a protein formulation according to the invention further comprises an excipient. In principle, any excipient is suitable that is compatible with relevant regulations for protein formulations are suitable for use in a protein formulation according to the invention. Preferably, a protein formulation according to the invention further comprises microcrystalline cellulose.

Preferably, a protein formulation comprising flour, comprises at least 1 wt.% of microcrystalline cellulose, more preferably at least 2 wt.%, at least 3 wt.% at least 4 wt.%, at least 5 wt.% of microcrystalline cellulose, based on the total dry weight of the protein formulation.

Preferably, a protein formulation comprising flour, comprises at most 30 wt.% of microcrystalline cellulose, more preferably at most 25 wt.%, at most 20 wt.% at most 18 wt.%, at most 15 wt.% of microcrystalline cellulose, based on the total dry weight of the protein formulation.

Preferably, a protein formulation comprising flour comprises between about 1 and about 30 wt.% of microcrystalline cellulose, more preferably between about 2 wt.% and about 25 wt.% of microcrystalline cellulose, between about 5 wt.% and about 20 wt.% of flour based on the total weight of the protein formulation.

Preferably, a protein formulation according to the invention comprises, based on total dry weight, between about 3 wt.% and about 25 wt.% of protective agent, preferably between about 5 wt.% and about 20 wt.% of protective agent, most preferably between about 10 wt.% and about 15 wt.% of protective agent, and between about 30 wt.% and about 70 wt.% of flour, preferably wheat flour, preferably between about 40 wt.% and about 60 wt.% of flour, preferably wheat flour and between about 5 wt.% and about 25 wt.% of microcrystalline cellulose, preferably between about 10 wt.% and about 20 wt.% of microcrystalline cellulose, more preferably between about 15 wt.% and about 18 wt.% of microcrystalline cellulose and between about 0.001 wt.% and about 50 wt.% of protein, more preferably between 0.1 wt.% and about 40 wt.% of protein, between about 1 wt.% and about 30 wt.%, between about 5 wt.% and about 20 wt.% of protein. Preferably, said protein is an enzyme, more preferably a phytase, chitase, cellulase or protease.

A protein formulation according to the invention may be in any form of shape. For example a protein formulation may be formulated as a solid, a liquid, an emulsion or suspension. Preferably, a protein formulation according to the invention is formulated as a solid, because this is the most dense form, can be stored efficiency and can be handled easily.

Preferably, a protein formulation according to the invention is formulated as a (pressed) tablet, a granulate, a powder, a capsule or a pellet.

Preferably, a protein formulation according to the invention is formulated as a mixture of the protein and the protective agent as defined herein. More preferably, said protein formulation is formulated as a mixture of solids to form a powder or granulate.

Preferably, the particle size of such a powder or granulate ranges between about 10 µm and about 3 mm, preferably between about 50 µm and about 2 mm, more preferably between about 100 µm and about 1 mm, between about 200 µm and about 900 µm, between about 350 µm and about 800 µm, most preferably between about 500 µm and about 75000 µm.

In a protein formulation according to the invention, the protein, preferably the enzyme and the protective agent as defined herein have been preferably at least substantially homogenously distributed in a protein formulation according to the invention. As used herein, the term "homogenous" is generally known to mean that the components are substantially evenly distributed throughout a protein formulation according to the invention. This may be tested by obtaining at least two samples from a protein formulation according to the invention, which should be substantially the same, e.g. have substantially the same composition and/or properties.

Preferably, the protective agent as defined herein is not present as a coating or said protein is not present as a core comprising a coating in a protein formulation according to the invention. Most preferably, a protein formulation according to the invention is not formulated as a core comprising a protein, at least partly coated with a protective agent as defined herein.

A solid protein formulation according to the invention may further optionally comprise a moisture-barrier and/or a gas-barrier coating, preferably comprising a sugar.

### (Food) product

The invention further relates to a food product, preferably animal feed, comprising the protein formulation according to the invention and one or more of a carbohydrate, a fat, and proteinaceous matter. In principle, the food product may be any food product that may benefit from the presence of a protein formulation according to the invention.

Any fat that is edible by animals including humans is suitable for use in the food product according to the invention. Such fats include animal fat, such as lard or butter, or a vegetable oil. Said fat may comprise saturated fatty acids, unsaturated fatty acids and polyunsaturated fatty acids and combinations thereof.

Examples of vegetable oils include rapeseed oil, sunflower oil, corn oil, soybean oil, coconut oil, palm oil, palm kernel oil, linseed oil, safflower oil, peanut oil, and olive oil.

The fat may further comprise one or more of an omega-3-fatty acid, such as eicosapentaenoic acid, docosahexaenoic acid and docosapentaenoic acid.

Any carbohydrate that is edible by animals including humans is suitable for use in the food product according to the invention, including digestible and indigestible carbohydrates.

Any proteinaceous matter that is edible by animals including humans is suitable for use in a food product according to the invention. For example, a protein or any part of a protein, such as non-hydrolyzed protein, native protein, hydrolyzed protein, peptides, such as oligopeptides, i.e. peptide comprising two to fifty amino acids, and free amino acids of any source may be used in the food product according to the invention. The total content of proteinaceous matter of a food product according to the invention is determinable with the Kjehldahl method as known in the art. As the skilled person will understand, the Kjehldahl method measure the total nitrogen content, hence to determine the protein content, the total content of protective agent needs to be subtracted.

Said food product may further comprise other food ingredients that are beneficial for humans including animals, such as vitamins and/or minerals.

Examples of minerals suitable for addition to a food product according to the invention are sodium, chloride, potassium, calcium, phosphorus, magnesium, sulfur, iron, zinc, iodine, selenium, copper, manganese, fluoride, chromium and/or molybdenum.

Examples of essential vitamins suitable for addition to a food product formulation according to the invention are vitamin A, vitamin B1 (thiamin), vitamin B2 (riboflavin), vitamin B3 (niacin), vitamin B5 (pantothenic acid), vitamin B6, vitamin B8 (biotin), vitamin B9, vitamin B10 (factor R), vitamin B11 (folate), vitamin B12, vitamin C, vitamin D, vitamin E and/or vitamin K.

Preferably, the food product is an animal feed. Most preferably, the food product relates to food for pet animals such as dog food, cat food, hamster food, guinea pig food, rodent food, mice food and rabbit food, or food for livestock such as horse food, cow food, pig food, goat food, sheep food, and poultry food, such as chicken, duck, goose, and turkey.

Said food product may be formulated in any form or shape, for example as a solid, such as in pellet form, tablet form, capsule form, powder form, or fluid form such as a gel, a suspension, an emulsion or a drink. Preferably, said food product is a solid, most preferably a pellet.

The invention further relates to a household product, preferably a detergent comprising a protein formulation according to the invention and one or more of a surfactant, chelating agent or a bleaching agent.

Said surfactant is preferably an anionic surfactant, such as a branched or a linear anionic surfactant, or a non-ionic surfactant such as a polyalkylene oxide condensate of alkyl phenols.

Said chelating agent may be any suitable chelating agent, such as an aluminosilicate material, a silicate, a polycarboxylate or a fatty acid.

Said bleaching agent may be any suitable bleaching agent, such as an oxygen bleach or an halogen bleach. In particular a bleaching agent that releases hydrogen peroxide, such as perborate or a hypohalite bleaching agent such as trichloroisocyanuric acid.

Said household product, preferably detergent, may optionally comprise one or more further components such as a foaming agent, a stabilizer, a solubilizer, a corrosion inhibitor, a dye transfer inhibitor, a fragrant, a brightener or a softener.

### Methods and uses

The invention further relates to a method for preparing a protein formulation according to the invention, comprising mixing a protein and a protective agent comprising (i) at least one amine and/or an ammonium group and (ii) at least one metal precipitating agent and/or a chelating molecule. Preferably said method further comprises mixing of one or more reducing sugars and/or metal ions, in particular one or more transition metal ions.

Said protein and said protective agent comprising (i) at least one amine and/or an ammonium group and (ii) at least one metal precipitating agent and/or a chelating molecule is as described herein above.

Mixing of said protein with said protective agent as defined herein may be performed using any method in the art, such as by whisking, beating or stirring. Preferably, a mixture of said protein, and said protective agent comprising (i) at least one amine and/or an ammonium group and (ii) at least one metal precipitating agent and/or a chelating molecule is obtained that is at least substantially homogeneous.

Preferably, said method further comprises a step of mixing water to form an aqueous mixture comprising a protein, a protective agent and preferably one or more reducing sugars and/or metal ions. Preferably, said aqueous mixture comprises at least 25 wt.% of water, more preferably at least 40 wt.% of water, at least 55 wt.% of water, most preferably at least 70 wt.% of water, based on the total weight of the aqueous mixture. Preferably, said aqueous mixture comprises between about 30 wt.% and about 70 wt.% of water, in particular between about 40 wt.% and about 60 wt.% of water, based on the total weight of the aqueous mixture.

Preferably, said method further comprises mixing a carrier, preferably a flour selected from the group consisting of wheat flour, barley flour, rye flour, spelt flour, triticale flour and combinations or derivative like starch thereof to obtain a protein formulation comprising flour.

Preferably, said method further comprising a step of mixing one or more excipients, in particular microcrystalline cellulose.

Typically, a protein formulation comprising flour and optionally microcrystalline cellulose is in the form of a dough, which is suitable for further processing such as extrusion, pelletizing, tableting and the like.

Preferably, a dough comprises a protein, preferably an enzyme, a protective agent, flour, water and microcrystalline cellulose.

In a preferred method according to the invention, said protein, said protective agent, said carrier, preferably said wheat flour, said water and optionally said excipient, preferably microcrystalline cellulose are subjected to a wet granulation process. In a wet granulation process, a mixture comprising protein, protective agent, carrier, preferably wheat flour and optionally excipient, preferably microcrystalline cellulose are mixed to form a dough, which is subsequently extruded in a suitable extruder, to form extruded dough particles, also referred to as pellets.

Regular wet granulation processes typically requires a large amount of energy to prepare a dough comprising protein and flour, which is not desirable from a durability and economical perspective. The inventor's surprisingly realized that the energy required to prepare a dough comprising protein and flour can be markedly reduced in presence of a salt. Furthermore, it is shown that a protective agent can be used, without outsalting a protein from a protein formulation according to the invention. This is shown in Examples 1 and 2 and Figures 1 and 2 of the present application.

This is advantageous, because a solid protein formulation, such as a pellet, can be advantageously prepared without requiring harsh manufacturing conditions, such as a high pressure, which may lead to degradation of a protein from a protein formulation.

Preferably, said moulding, shaping, pressing, tabletting, pelleting or extruding occurs at a temperature of below 60 °C, more preferably below 50 °C, below 45 °C, below 40 °C, below 25 °C, most preferably below 20 °C. In particular, said moulding, shaping, tabletting, pelleting, pressing or extruding occurs at a temperature between 10 °C and 60 °C, more preferably between 20 and 50 °C, even more preferably between 30 and 45 °C.

Preferably, said pressing, tabletting, pelleting or extruding occurs at a pressure of less than 40 bar, more preferably less than 30 bar, less than 20 bar, less than 15 bar, less than 10 bar, most preferably less than 5 bar.

Preferably, said pressing, tabletting, pelleting or extruding occurs at a pressure of between about 0.5 and about 40 bar, more preferably between about 1 and about 25 bar, between about 2 and about 20 bar, between about 3 and about 15 bar, most preferably between about 4 and about 10 bar, in particular between about 5 and about 7 bar.

At such pressures, it was advantageously found that a heat stable protein formulation could be obtained at technical or industrial scale. Without wishing to be bound by any theory, it is believed that this may be due to reduced degradation of the protein by mechanical processing of the protein formulation according to the invention.

The invention further preferably relates to a method for preparing a protein formulation further comprising a step of spray drying a mixture comprising a protein, preferably an enzyme, a protective agent as defined herein, preferably one or more reducing sugars and/or metal ions, preferably transition metal ions and optionally a carrier, preferably a flour, to obtain a spray dried protein formulation.

Preferably, said mixture further comprises water, more preferably at least 20 wt.%, at least 30 wt.% of water, more preferably at least 40 wt.% of water, at least 50 wt.% of water, at least 60 wt.% at least 70 wt.%, at least 80 wt.%, most preferably at least 90 wt.% of water, based on the total weight of the mixture.

Preferably, said mixture comprises between about 70 wt.% and about 99 wt.% of water, in particular between about 80 wt.% and about 95 wt.% of water, based on the total weight of the mixture.

A method according to the invention further preferably comprises a step of drying a protein formulation or food product according to the invention. Said drying may be achieved using any suitable method known in the art, such as by placing said protein formulation or food product in an oven. Preferably said protein formulation or food product is dried at a temperature of at least 40 °C, preferably at least 50 °C, most preferably at least 60 °C. Typically, a protein formulation or food product is dried at a temperature of between 40 °C and 80 °C, preferably around 60 °C.

Preferably, a dried protein formulation according to the invention, comprises less than 10 wt.% of water, based on the total weight of the dried protein formulation, preferably less than 5 wt.% of water, more preferably less than 4 wt.% of water.

Preferably, a dried protein formulation according to the invention, comprises between about 0.5 wt.% and 10 wt.% of water, preferably between about 1 wt.% and about 5 wt.% of water, more preferably between about 2 wt.% and about 4 wt.% of water, based on the total weight of the dried protein formulation.

Preferably, a method according to the invention does not comprise a step of coating a core comprising a protein with a coating comprising or substantially consisting of a protective agent as defined herein.

The invention further relates to a method of preparing a food product, preferably animal feed, comprising mixing a protein formulation according to the invention with at least one fat, protein or carbohydrate.

Preferably, said mixing comprises a step of moulding, shaping, pressing, tabletting, pelleting or extruding the mixture of a protein formulation according to the invention and one or more of a carbohydrate, a fat and proteinaceous matter into a desired form. Preferably, said protein formulation according to the invention is extruded to form a granulate.

Preferably, said moulding, shaping, pressing, tabletting, pelleting or extruding occurs at an elevated temperature, preferably a temperature of above 40 °C, more preferably above 50 °C, above 60 °C, above 70 °C, above 75 °C, most preferably above 80 °C. In particular, said moulding, shaping, tabletting, pelleting, pressing or extruding occurs at a temperature between 50 °C and 150 °C, more preferably between 60 and 130 °C, even more preferably between 80 and 120 °C.

Preferably, said moulding, shaping, pressing, tabletting, pelleting or extruding takes place for a duration about 10 seconds to about 5 hours, preferably between about 20 seconds to about 3 hours, between about 30 seconds to about 2 hours, between about 45 seconds to about 1 hours, between about 1 minute to about 45 minutes, between about 1.5 minute and about 30 minutes, most preferably between about 2 minutes and about 15 minutes.

It has been advantageously found that a protein formulation according to the invention is substantially stable under these conditions. Preferably at such temperatures, at least at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, most preferably 100% of the enzyme is still active.

The invention further relates to a method for feeding an animal, comprising providing a food product or protein formulation, preferably enzyme formulation according to the invention and administering said food product or protein formulation, preferably enzyme formulation to an animal in need thereof.

The food product or protein formulation may be administered to any animal that benefits from administration of the protein formulation. Preferably, the food product or protein formulation is administered to livestock, such as cattle, goat, sheep, lamb, pig and horse, or to poultry, such as chicken, duck, goose, turkey, pheasant and fowl or to a pet animals such as cat, dog, rabbit, guinea pig, hamster or rat, or to wild animals such as deer, haze or wild boar.

The invention further relates to the use of a protective agent, wherein the protective agent comprises (i) at least one amine and/or an ammonium group and (ii) at least one metal precipitating agent and/or a chelating molecule, preferably triammonium citrate, diammonium hydrogen citrate, ammonium dihydrogen citrate, ammonium dihydrogen phosphate, ammonium phosphate, diammonium hydrogen phosphate, ammonium dihydrogen phosphate and diammonium tartrate and ammonium hydrogen tartrate in improving the stability of a protein formulation, in particular in improving the tolerance against reducing sugars and/or metal ions, heat-stability, and/or storage stability of a protein formulation.

For the purpose of clarity and a concise description, features are described herein as part of the same or separate embodiments, however, it will be appreciated that the scope of the invention may include embodiments having combinations of all or some of the features described.

The invention is illustrated by the following non-limiting examples.

### Example 1

300 g wheat flour (Type 405) was mixed with 168 g tap water and with 0-6 wt.% of ammonium sulphate, sodium chloride, sodium sulphate, ammonium citrate and ammonium acetate. The power input into the mixing process were measured by the Farinograph (Farinograph E, Brabender) in Farinograph Units (FE), wherein 1 FE corresponds to 9.8 ± 0.2 mN·m.

The results are shown in Figure 1. It can be derived from Figure 1 that the presence of a salt reduces the energy demand required to knead a dough. This would reduce the energy demand for a wet granulation process and allows the use of less harsh manufacturing conditions, thereby reducing the chance of degradation of the enzyme present in an enzyme formulation.

### Example 2

300 g of various types of flour and starch was mixed 168 g of tap water and with 0-6 wt.% of ammonium sulfate. The power input into the mixing process were measured by the Farinograph (Farinograph E, Brabender) in Farinograph Units (FE), wherein 1 FE correspond to 9.8 ± 0.2 mN·m.

The results are shown in Figure 2. It can be derived from Figure 2 that the presence of ammonium sulphate reduces the energy demand required to knead a dough, with exception of pure starch. This may be explained by the fact that starch does not contain the gluten fraction of flour.

### Example 3: Testing stability of various enzyme formulations using different potential protective agents

### Manufacturing of phytase pellets

In a food processor (MUM 2, Bosch) dry ingredients (wheat flour, microcrystalline cellulose and salt) were homogenized for one minute. Subsequently, a 6-phytase solution (Enzy Phostar, Kaesler Nutrition GmbH) with ≥ 40.000 U/g was added to each prepared batch and the mixture was kneaded for about 3-4 minutes until the dough started to separate into individual small balls. The final contents of the dough is shown in Table 1 for triammonium citrate. The examples were repeated using sodium chloride, ammonium acetate, ammonium chloride, ammonium sulphate, diammonium citrate, diammonium hydrogen phosphate, diammonium tartrate and ammonium acetate instead of triammonium citrate. The contents of salt and phytase may vary slightly to obtain the desired consistency of the dough, as described herein above.

**Table 1; composition of dough comprising triammonium citrate; * based on the total weight of the dry ingredients.**

| Triammonium citrate (wt.%)* | Triammonium citrate (g) | Flour (g) | MCC (g) | Phytase solution (Unit) |
|---|---|---|---|---|
| 0.0 | 0 | 220 | 57 | 7,401,584 |
| 8.4 | 27.7 | 220 | 57 | 8,310,692 |
| 12.0 | 41.55 | 220 | 57 | 8,610,375 |
| 15.5 | 55.4 | 220 | 57 | 8,342,872 |
| 18.8 | 69.25 | 220 | 57 | 7,769,652 |
| 22.0 | 83.1 | 220 | 57 | 7,039,550 |
| 24.9 | 96.95 | 220 | 57 | 6,782,908 |

Then the batch was removed from the bowl and the mincer attachment was installed with a 1 mm die. The dough was passed through the mincer at level 2 and the extruded strings were collected. Subsequently, the strings were rounded in the spheronizer (MBS, Caleva) at 1600 rpm and a residence time of 30 seconds. The obtained pellets were dried on a plastic tray in the heating oven (Heratherm OMH400, Thermo Scientific) at 60°C for 4 hours. The measurement of the residual moisture of the pellets was measured at 103°C with an automatic stop (Sartorius MA35). Salt contents were determined based on the dry weight of the phytase pellet.

### Stability test of the pellets

For each condition, two samples of 0.5 grams of the phytase pellets were prepared into vials. One vial was left at room temperature as a control (determination of enzyme start activity), the other sample was placed in an autoclave (VX-150, Systec GmbH) for the thermostability stress test and autoclaved open with defined parameters (determination of the residual (relative) enzyme activity). After starting the autoclave, it heated up to a temperature of 94°C and this temperature was maintained for two minutes. This allowed the samples to react with the hot steam to simulate a conditioning step as needed for a feed pelleting process. Afterwards, the device was opened after cooling down to 74°C and the samples were placed directly on ice and further cooled down for 30 minutes.

For enzyme extraction, 100 ml of a 250 mM HAc-NaAc buffer pH 5.5 with 0.1 % polysorbate 20 (v/v) (reaction buffer) was added to all samples and then stirred at 200 rpm for 45 minutes. The samples were diluted again with the same extraction buffer so that the absorbances to be measured were in the linear range of the plate photometer. The activity of 6-phytase in the samples was determined by using the ISO method 30024:2009. The determination of the activity is based on the detection of the released phosphate from the substrate sodium phytate (68388, Sigma-Aldrich), which in turn reacts in nitric acid solution with molybdate and vanadate to form a yellow complex that was detected at an optical density of 415 nm in a plate photometer (FLUOstar Omega, BMG LABTECH GmbH). The samples were measured as duplicates ± SD. The relative activities of the heated samples were determined and the non-heated samples were taken as 100% reference.

### Results

The results are shown in Figure 3. It can be derived from Figure 3 that the activity of 6-phytase was not improved in presence of increasing concentrations of sodium chloride, ammonium acetate, ammonium chloride or ammonium sulphate, and was even reduced in presence of increasing concentrations of ammonium acetate.

Without being bound by theory, it is believed that in the 6-phytase formulation comprising ammonium acetate, the 6-phytase fraction was at least partly salted out of the formulation. As a result, the 6-phytase was exposed to mechanical stress of the food processor which lead to further degradation of 6-phytase.

However, in samples comprising triammonium citrate, diammonium hydrogen acetate, diammonium tartrate and Na₂HPO₄, the stability of 6-phytase was substantially improved. As can be derived from Figure 3, there is a linear correlation between the concentration of ammonium salt and relative activity of 6-phytase.

The results demonstrate that enzyme formulations comprising protective agent as defined herein exhibit improved activity compared to enzyme formulations lacking protecting agent.

### Example 4: Testing stability of various enzyme formulations using different enzymes

### Manufacture of different enzyme pellets

In the food processor (MUM 2, Bosch) the dry components (wheat flour, microcrystalline cellulose and triammonium citrate) were homogenized for one minute. 30 ml of a cellulase TXL solution (Trichoderma longibrachiatum, ASA Spezialenzyme GmbH) with > 30 U/ml. The final contents of the dough is shown in Table 2 for triammonium citrate and Table 3 for sodium chloride. The examples were repeated using different enzyme solutions (or 20 ml of a protease SO2 solution (*Aspergillus niger*, ASA Spezialenzyme GmbH) or 5 ml of a chitinase solution (*Trichoderma harzianum,* ASA Spezialenzyme GmbH) with > 100 U/ml were added to each prepared batch, respectively). The content water may vary slightly to obtain the desired consistency of the dough, as described herein above.

**Table 2; composition of dough comprising triammonium citrate; * based on the total weight of the dry ingredients. One Unit of activity is defined as the amount of enzyme required to release one micromole of glucose reducing sugar equivalents per minute from barley β-glucan (10 mg/ mL) at pH 4.5 and 40°C.**

| Triammonium citrate (wt.%)* | Triammonium citrate (g) | Flour (g) | MCC (g) | Cellulase TXL (Units) | Municipal water (g) |
|---|---|---|---|---|---|
| 5.2 | 13,85 | 220 | 57 | 900 | 153.7 |
| 9.9 | 27,7 | 220 | 57 | 900 | 150.6 |
| 17.9 | 55,4 | 220 | 57 | 900 | 135.2 |
| 24.7 | 83,1 | 220 | 57 | 900 | 121.5 |

**Table 3; composition of dough comprising sodium chloride; * based on the total weight of the dry ingredients. One Unit of activity is defined as the amount of enzyme required to release one micromole of glucose reducing sugar equivalents per minute from barley β-glucan (10 mg/ mL) at pH 4.5 and 40°C.**

| Sodium chloride (wt.%)* | Sodium chloride (g) | Flour in [g] | MCC in [g] | Cellulase TXL ( Units) | Municipal water [g] |
|---|---|---|---|---|---|
| 5.2 | 13,85 | 220 | 57 | 900 | 153.7 |
| 9.9 | 27,7 | 220 | 57 | 900 | 150.6 |
| 17.9 | 55,4 | 220 | 57 | 900 | 135.2 |
| 24.7 | 83,1 | 220 | 57 | 900 | 121.5 |

The mixture was kneaded for about 3-4 minutes until the finished dough begins to separate into individual small balls. Water was optionally added to allow formation of such individual balls. Then the batch was removed from the bowl and the mincer attachment was installed with a 1mm die. The dough was passed through the mincer at level 2 and the extruded strings were collected. Subsequently, the strings were rounded in the spheronizer (MBS, Caleva) at 1600 rpm and a residence time of 30 seconds. The obtained pellets were dried on a plastic tray in a heating oven (Heratherm OMH400, Thermo Scientific) at 60 °C for 4 hours. The measurement of the residual moisture was measured at 103 °C with an automatic stop (Sartorius MA35). Finally, the pellets had a residual moisture content of < 10%. Ideally < 5%. Salt contents were determined based on the dry weight of the enzyme pellet.

### Stability test of the pellets

For the analysis each condition and enzyme, two identical samples of 1 g were weighed into vials. One vial was left at room temperature as a control (determination of enzyme start activity), the other was placed in an autoclave (VX-150, Systec GmbH) for the thermostability stress test and autoclaved open with defined parameters (determination of the residual (relative) enzyme activity). After starting the autoclave, it heated up to a temperature of 94°C and this temperature was maintained for two minutes. This allowed the samples to react with the hot steam to simulate a conditioning step as needed for a feed pelleting process. Afterwards, the device was opened after cooling down to 74°C and the samples were placed directly on ice and further cooled down for 30 minutes.

For cellulose enzyme extraction, 50 ml of a 25 mM HAc-NaAc buffer pH 4.5 was added to all samples and then stirred at 200 rpm for 45 minutes. Then 1.5 ml of the extract was taken and centrifuged at 10,000 xg for 10 minutes. If necessary to stay within the linear measuring range of the photometer, the samples was diluted with the sodium acetate buffer. To analyse the activities of cellulase TXL, which possess endo-1,4-beta-glucanase activity, the assay manual of beta-glucazyme tablets was followed (The substrate employed is Azurine-crosslinked barley β-glucan; T-BGZ-200T; Megazymes) according to the manufacturer's instructions. The diluted or undiluted clarified extracts in a 25 mM HAc-NaAc buffer pH 4.5 were each preheated to 500 µl for 5 minutes in a glass test tube at 40°C. Then the enzymatic reaction was started by adding a beta-glucazyme tablet. After 10 minutes the reaction was stopped by adding 10.0 mL of Trizma Base solution (2 % w/v, pH 8.5). After the samples have cooled down for 5 minutes at room temperature, they were filtered using Whatmann filters and 300 µL of the clarified solution was analysed in a plate photometer (FLUOstar Omega, BMG LABTECH GmbH, Germany). The absorbance is measured at 590 nm. The samples were measured as duplicates ± SD. The relative activities of the heated samples were determined and the non-heated samples were taken as 100% reference.

For protease extraction, 100 ml of a 25 mM HAc-NaAc buffer pH 4.0 (reaction buffer) was added to all samples and then stirred at 200 rpm for 45 minutes. Then 1.0 ml of the extract was taken and centrifuged at 10,000 xg for 10 minutes. If necessary to stay within the linear measuring range of the photometer, the samples were diluted with the sodium acetate buffer. To analyse the activities of Protease S-02 which possess an acidic peptidase activity, the assay manual of the Pierce^{™} Colorimetric Protease Assay Kit (Thermo Scientific, 23263) was followed. A modulation of the assay for acidic peptidases was performed (according to manual instructions). For this, 50 µl of the succinylated casein (2 mg/ml reaction buffer) in reaction buffer was pipetted into the wells. 25 µl of the diluted or non-diluted samples or controls are then added to the substrate. The microtitre plate was incubated at 40°C for 20 minutes. 75 µl of a 50 mM sodium borate buffer (pH 8.5) was then pipetted to the samples to stop the enzymatic reaction. Then 50 µl of TNBSA solution (2,4,6-trinitrobenzene sulfonic acid, 5% (w/v) in methanol) was pipetted to the samples and incubated at RT for 20 minutes. The absorbance was analyzed at 450 nm in a plate photometer (FLUOstar Omega, BMG LABTECH GmbH). The samples were measured as duplicates ± SD. The relative activities of the heated samples were determined and the non-heated samples were taken as 100% reference.

For chitinase extraction, 10 ml of a 100 mM HAc-NaAc buffer pH 5.5 (reaction buffer) was added to all samples and then stirred at 200 rpm for 45 minutes. Then 1.0 ml of the extract was taken and centrifuged at 10,000 xg for 10 minutes. The clear supernatant was directly used for enzyme activity assay. The chitinase activity detection is based on the enzymatic hydrolysis of chitinase substrates (the 1,4-beta-poly-N-acetylglucosaminidase activity on the specific substrate 4-Nitrophenyl-N-acetyl-beta-glucosaminide (Carl Roth GmbH + Co. KG, Germany)) using a chitinase assay kit (Sigma Aldrich; CS0980) according to the manufacturer's instructions. This hydrolysis releases p-nitrophenol (4-nitrophenol), which can be measured colorimetrically after ionisation in basic pH value at 405 nm and was used as a standard to test the linear range in the plate photometer. 90 µl of the substrate solution (1 mg/ml in bidistilled H2O) was placed in 96-well plates. To this was added 10 µl of the extracts or standards. After mixing, incubation at 37 °C for 30 minutes was followed by addition of 200 µl of a 112.5 mg/ml (w/v %) sodium carbonate decahydrate solution. The absorbance was analyzed at 405 nm plate photometer (FLUOstar Omega, BMG LABTECH GmbH, Germany). The samples were measured as duplicates ± SD. The relative activities of the heated samples were determined and the non-heated samples were taken as 100% reference.

### Results

The results are shown in Figure 4. As can be seen, for each enzyme, stability was improved in presence of tribasic ammonium citrate, but not in presence of sodium chloride.

### Example 5: Manufacturing of enzyme formulation according to the invention at technical scale

Dry components (440 g wheat flour, 114 g microcrystalline cellulose and varying amounts of triammonium citrate) were homogenized for one minute in a bag by hand. The different mixtures were poured separately into a feeder (K-ML-SFS-KT20, Coperion K-Tron). The dry mixtures were then fed into an extruder at a dosage of 1 kg/h (ZSE18MAXX-HP, Leistritz). The screw speed was adjusted at 500 rpm. The screw design consists mainly of conveying elements and the dry product addition success in zone 6 and the liquid addition in zone 7. The discharge of the extruder was actively cooled with tap water (approx. 14°C). Phytase solution with ≥ 40.000 FTU/g (ENZY Phostar, Kaesler Nutrition GmbH) was added via a pump (NEMA 4x IP66, Watson Marlow) with 1 mm liquid nozzle and a dosage of 7.5 to 13 g/min, depending on the formulation of dry components to from regular extrudate at moderate pressure in the extruder. The composition of the dough samples is given in Table 4. The dough was extruded through a die with a diameter of 0.8 mm and subsequently rounded in the spheronizer (MBS, Caleva) at 1600 rpm and a residence time of 30 seconds. The obtained pellets were dried in the drying oven (Heratherm OMH400, Thermo Scientific) at up to 60 °C product temperature. The measurement of the residual moisture was measured at 103 °C with an automatic stop (MA35, Sartorius). Triammonium citrate contents were determined based on the dry weight of the phytase pellet.

**Table 4; composition of dough comprising sodium chloride; * based on the total weight of the dry ingredients.**

| Triammonium-citrate (wt.%)* | Triammonium citrate (g) | Flour in [g] | MCC in [g] | Phytase (ca. 40.000 units/g) in [g/h] |
|---|---|---|---|---|
| | Mixture dosed with 1kg/h | | | |
| 0.9 | 5.54 | 440 | 114 | 708 |
| 0.9 | 5.54 | 440 | 114 | 798 |
| 1.8 | 11.08 | 440 | 114 | 690 |
| 1.8 | 11.08 | 440 | 114 | 792 |
| 4.3 | 27.7 | 440 | 114 | 768 |
| 4.4 | 27.7 | 440 | 114 | 708 |
| 4.4 | 27.7 | 440 | 114 | 702 |
| 8.3 | 55.4 | 440 | 114 | 708 |
| 8.3 | 55.4 | 440 | 114 | 738 |
| 8.4 | 55.4 | 440 | 114 | 678 |
| 12.0 | 83.1 | 440 | 114 | 690 |
| 12.0 | 83.1 | 440 | 114 | 672 |
| 15.4 | 110.8 | 440 | 114 | 654 |
| 15.5 | 110.8 | 440 | 114 | 624 |
| 18.8 | 138.5 | 440 | 114 | 558 |
| 22.2 | 166,2 | 440 | 114 | 486 |
| 24.9 | 166.2 | 440 | 114 | 450 |

In a second run, the experiment was repeated at different pressures (varying from 0-30 bar as indicated by a pressure sensor). The pressure was changed by varying the content of enzyme solution and/or the content of salt. As can be derived from Figure 5b, by keeping the pressure low, preferably below 10 bar or even lower, an enzyme formulation could be prepared with minimal loss of enzyme due to degradation.

### Example 6: Manufacturing of an enzyme formulation according to the invention using spray drying.

An enzyme formulation was prepared by mixing 30 g wheat flour, 10.5 g of triammonium citrate, 20 g of fermentation supernatant (*E.coli* 6-phytase with 146 FTU/ml) and 139.5 g of tap water. Subsequently the homogenized mixture was spraydried using a Büchi B-290 Advanced, with a nozzle diameter of 1.5 mm at a pressure of 8 bar and using the following settings:
Spray parameters: Inlet temp.: 110 °C
Outlet temp.: 70 °C
Pump capacity: 20
Flow: 40 mm
Aspirator capacity: 100
Collecting vessel temp.: 46-48 °C
Spraying time: 15 min.

A reference enzyme formulation (lacking ammonium citrate) was prepared by mixing 30 g wheat flour, 20 g of fermentation supernatant (*E.coli* 6-phytase with 146 FTU/ml) and 150 g of tap water. Subsequently the homogenized mixture was spraydried using a Büchi B-290 Advanced, with a nozzle diameter of 1.5 mm at a pressure of 8 bar and using the following settings:
Spray parameters: Inlet temp.: 110 °C
Outlet temp.: 66 °C
Pump capacity: 20
Flow: 40 mm
Aspirator capacity: 100
Yield: 2.42 g
Collecting vessel temp.: 40-46 °C
Spray time: 15 min.

The obtained powders were subsequently subjected to heat and the relative activity was determined according to the procedures described in Example 3. The results are shown in Figure 6.

As can be derived from Figure 6, a powdered enzyme formulation according to the invention was more stable against heat compared to a reference enzyme formulation. These results indicate that an enzyme formulation according to the invention may also be prepared by means of spray-drying.

### Example 7: Stability of enzyme formulation according to the invention in presence of glucose.

In a food processor (MUM 2, Bosch) the dry components (79,5 g wheat flour, 20,6 g microcrystalline cellulose, 35 g tribasic ammonium citrate and 0-4 wt.% of glucose) were homogenized for one minute. 50 - 65 grams of a 6-phytase solution (Enzy Phostar, Kaesler Nutrition GmbH) with ≥ 40.000 U/g was added to each prepared batch and the mixture was kneaded for about 3-4 minutes until the finished dough begins to separate into individual small balls. The finished batch was removed from the bowl and the mincer attachment was installed with a 1mm die. The dough was passed through the mincer at level 2 and the extruded strings are collected. Subsequently, the strings were rounded in the spheronizer at ~ 1600 rpm and a residence time of ~30 seconds. The obtained pellets were dried on a plastic tray in a heating oven at 60 °C for 4 hours. The measurement of the residual moisture is measured at 103 °C with an automatic stop (Sartorius MA35).

The obtained pellets were subsequently subjected to heat and the relative enzyme activity was determined according to the procedures described in example 3. The results are shown in Figure 7.

As can be derived from Figure 7, in presence of additional glucose, the relative phytase activity was reduced. It is envisaged that in presence of glucose, more amino acids present in the enzyme participate into the Maillard reaction and therefore more degradation is observed. These results indicate that the Maillard reaction is the underlying cause of the observed lack of stability when heat is applied.

### Example 8: Stability of enzyme formulation according to the invention in presence of iron (III) chloride.

In a food processor (MUM 2, Bosch) the dry components (79,5 g wheat flour, 20,6 g microcrystalline cellulose and 35 g tribasic ammonium citrate) were homogenized for one minute. 50 - 65 grams of a 6-phytase solution (Enzy Phostar, Kaesler Nutrition GmbH) with ≥ 40.000 U/g was added and the mixture was kneaded for about 3-4 minutes until the finished dough begins to separate into individual small balls. In addition, a control formulation was prepared lacking the triammonium citrate, using the same protocol. The control formulation contained 79.5 g wheat flour, 20.6 g microcrystalline cellulose and 50-65 g 6-phytase solution (Enzy Phostar, Kaesler Nutrition GmbH) with ≥ 40.000 U/g).

The finished batches were removed from the bowl and the mincer attachment was installed with a 1mm die. The dough was passed through the mincer at level 2 and the extruded strings are collected. Subsequently, the strings were rounded in the spheronizer at ~1600 rpm and a residence time of ~30 seconds. The obtained pellets were dried on a plastic tray in a heating oven at 60 °C for 4 hours. The measurement of the residual moisture is measured at 103 °C with an automatic stop (Sartorius MA35).

The obtained pellets were subsequently subjected to heat and the relative enzyme activity was determined according to the procedures described in example 3. The results are shown in Figure 8.

As can be derived from Figure 8, in presence of additional iron (III) ion, the relative phytase activity was reduced. It is envisaged that in presence of Fe³⁺, more amino acid residues present in the enzyme participate into the Maillard reaction and therefore more degradation is observed. These results suggest that the Maillard reaction is the underlying cause of the observed lack of stability when heat is applied. In the presence of triammonium citrate the slope of graph is slower and less degradation is observed. The results indicate that triammonium citrate acts as a protective agent by chelating metal ions.

## Claims

1. A protein formulation comprising a protein and a protective agent, said protective agent comprising (i) at least one amine and/or an ammonium group and (ii) at least one metal precipitating agent and/or chelating molecule.

2. The protein formulation according to claim 1, wherein the protective agent comprises an ammonium salt.

3. The protein formulation according to any one of the preceding claims, wherein the protective agent comprises a chelating molecule.

4. The protein formulation according to claim 3, wherein the chelating molecule is selected from citrate and tartrate, preferably triammonium citrate, diammonium hydrogen citrate and diammonium tartrate.

5. The protein formulation according to any one of the preceding claims, wherein the protective agent comprises a metal precipitating agent, preferably a phosphate, a carbonate or a sulfide, more preferably triammonium phosphate, diammonium hydrogen phosphate, ammonium dihydrogen phosphate, ammonium carbonate, ammonium hydrogen carbonate or ammonium hydrogen sulfide.

6. The protein formulation according to any one the preceding claims, further comprising one or more reducing sugars, preferably one or more reducing sugars that is/are at least partly produced by a microorganism, even more preferably wherein the microorganism is a micro-organism that has expressed the enzyme or wherein the one or more reducing sugars is/are present as remnant(s) from a culture medium.

7. The protein formulation according to any one the preceding claims, further comprising a metal ion, preferably transition metal ion, more preferably a zinc ion, an iron ion, a cobalt ion, a manganese ion or a molybdenum ion, even more preferably wherein said transition metal ion is present as remnant from a culture medium.

8. The protein formulation according to any one of the preceding claims, comprising a carrier, preferably a flour selected from wheat flour, barley flour, rye flour, spelt flour, triticale flour and combinations thereof, preferably wherein the formulation comprises wheat flour.

9. The protein formulation according to any one of the preceding claims, wherein the protein is an enzyme, preferably wherein the enzyme is selected from phytase, chitase, peptidase and cellulase.

10. The protein formulation according to any one of the preceding claims, wherein the protective agent is present in an amount of between about 5 wt.% and about 50 wt.%, preferably between about 10 wt.% and about 40 wt.%, most preferably between about 15 wt.% and about 30 wt.%, based on dry weight of the protein formulation.

11. A food product comprising the protein formulation according to any one of the preceding claims and at least one fat and/or further carbohydrate and/or further protein.

12. A method for preparing a protein formulation according to any one of claims 1 to 10, comprising mixing a protein and a protective agent comprising (i) at least one amine and/or an ammonium group and (ii) at least one metal precipitating agent and/or a chelating molecule to obtain a protein formulation according to any one of claims 1 to 10.

13. The method according to claim 12, further comprising mixing a carrier, preferably a flour selected from wheat flour, barley flour, rye flour, spelt flour, triticale flour and combinations thereof to obtain the protein formulation comprising a carrier, preferably flour.

14. The method according to claim 12 or claim 13, further comprising spray-drying said protein formulation to obtain a spray-dried protein formulation.

15. The method according to claim 13, further comprising extruding said protein formulation comprising flour to obtain an extruded protein formulation, preferably wherein the pressure during said extruding is between 0.5 and 40 bar, preferably between 1 and 20 bar, in particular between 2 and 5 bar.

16. Use of a protective agent, wherein the protective agent comprises (i) at least one amine and/or an ammonium group and (ii) at least one metal precipitating agent and/or a chelating molecule, preferably wherein the protective agent is triammonium citrate, diammonium hydrogen citrate, ammonium dihydrogen citrate, triammonium phosphate, diammonium hydrogen phosphate, ammonium dihydrogen phosphate, ammonium hydrogen tartrate and diammonium tartrate, in improving the stability of a protein formulation, in particular in improving the heat-stability of a protein.
